# EUROPÄISCHE PATENTANMELDUNG

(11) **EP 2 565 598 A2**
(43) Veröffentlichungstag der Anmeldung: **06.03.2013**
(21) Anmeldenummer: 12181216.8
(22) Anmeldetag: 21.08.2012
(51) Int. Cl.: G01G 19/50, A61B 5/053

(54) **Personenwaage**

(30) Priorität: 31.08.2011 DE 102011053155
(71) Anmelder: Leifheit AG, 56377 Nassau (DE)
(72) Erfinder: Stange, Dr., Pedro, 65582 Diez (DE)
(74) Vertreter: Bungartz, Klaus Peter

(57) **Zusammenfassung**

Die Erfindung betrifft eine Personenwaage mit einer Wägeplatte (1) zur Aufnahme einer zu wiegenden Last einer auf der Wägeplatte stehenden Person (2), einer von mindestens einen physiologischen Sensor (3) gebildeten Sensorik zur Messung wenigstens einer physiologischen Messgröße des auf der Wägeplatte (1) stehenden Benutzers (2) und einer Auswerteelektronik, die anhand einer Deformation von Wägezellen nach einer Messperiode das Gewicht der Person zu bestimmen und über eine Ausgabevorrichtung auszugeben vermag.

Es sind Personenwaagen mit einer Messung des Körperfettanteils am Gesamtgewicht eine Person bekannt. Diese Waagen haben den Nachteil, dass sie nur eine starre physiologische Messgröße auszugeben vermögen. Dies ändert die Erfindung dadurch, dass die Auswerteelektronik die zeitliche Änderung der physiologischen Messgröße während der Messperiode über eine Ausgabevorrichtung (4) auszugeben vermag.

## Beschreibung

### Technisches Gebiet

Die Erfindung betrifft eine Personenwaage mit einer Wägeplatte zur Aufnahme einer zu wiegenden Last einer auf der Wägeplatte stehenden Person, einer von mindestens einen physiologischen Sensor gebildeten Sensorik zur Messung wenigstens einer physiologischen Messgröße des auf der Wägeplatte stehenden Benutzers und einer Auswerteelektronik, die anhand einer Deformation von Wägezellen nach einer Messperiode das Gewicht der Person zu bestimmen und über eine Ausgabevorrichtung auszugeben vermag.

### Stand der Technik

Derartige Personenwaagen sind zum Beispiel aus der DE 20 2005 012714 U1 bekannt. Diese Personenwaage weist eine Wägeplatte auf, an deren Oberseite Elektroden vorgesehen sind, die zur Messung des Körperfettanteils verwendet werden. Hierzu wird der elektrische Widerstand innerhalb eines Stromkreises, der vor einer ersten Elektrode über den Fuß der zu wiegenden Person, durch die Beine dieser Person zu einer zweiten Elektrode an der Wägeplatte verläuft, gemessen und ausgewertet. In Abhängigkeit des Wiegeergebnisses wird dann der Anteil des Körperfetts über den elektrischen Widerstand bestimmt und über die Ausgabevorrichtung zusammen mit dem Gewicht angezeigt. Dabei ist hier die Ausgabevorrichtung von der Waage unabhängig und weist einen Speicher für die gemessenen Werte auf.

Aus der JP 2005-077124 A ist eine Waage bekannt, die den Body-Mass-Index errechnet und dem Benutzer einen Wert zurückgibt, der die Änderung desselben darstellt. Hier wird der Benutzer über den zeitlichen Verlauf des Messergebnisses über mehrere Messungen hinweg unterrichtet.

Aus der DE 10 2009 049 567 A1 ist wiederum eine Waage bekannt, bei der der Verlauf der Bewegung des Schwerpunktes des Benutzers gemessen und angezeigt wird. Hierdurch kann der Benutzer zur Verbesserung der Messgenauigkeit ruhig auf der Waage stehen.

Obwohl die bekannten Waagen zuverlässig das Gewicht und den Körperfettanteil als eine mögliche physiologische Größe der zu wiegenden Person bestimmen und anzeigen können, haben sie doch den Nachteil, dass sie sich auf die Messung eines momentanen Zustandes beschränken. Insbesondere kann der Benutzer die bekannten Waagen nicht dazu benutzen, um physiologische Größen kurzfristig während des Stehens auf der Waage zu beeinflussen.

### Kurzbeschreibung der Erfindung

Aufgabe der Erfindung ist es daher, eine Personenwaage zu schaffen, die während der Messperiode einen Trainings-oder Entspannungseffekt für die Person bietet.

Diese Aufgabe wird nach der Erfindung dadurch gelöst, dass die Auswerteelektronik die zeitliche Änderung der physiologischen Messgröße während der Messperiode über eine Ausgabevorrichtung in einer Weise auszugeben vermag, dass die Person diese wahrzunehmen vermag, um die Person so in die Lage zu versetzen, durch die Wahrnehmung der zeitlichen Änderung der physiologischen Messgröße im Sinne einer Rückkopplung ihre, die physiologische Messgröße beeinflussenden Körperfunktionen bewusst zu beeinflussen.

Erfindungsgemäß wird es nun möglich, die üblicherweise bei einer Personenwaage ohnehin vorhandene Sensorik zu nutzen, um dieser Waage eine zusätzliche Funktion hinzuzufügen. Der Benutzer erhält aufgrund der Tatsache, dass die Auswerteelektronik die zeitliche Änderung einer seiner von der physiologischen Messgröße wiedergegebenen Körpereigenschaft während der Messedauer an ihn ausgibt, eine Rückkopplung im Rahmen eines Biofeedbacks über den Verlauf der Messgröße während er auf der Wägeplatte steht. Die Person nimmt also auf irgendeine Weise die Änderung des Signals, dass einer oder mehreren physiologischen Messgrößen entspricht, wahr und kann, insbesondere auch über eine Anleitung hierzu angeregt, Körperfunktionen oder -haltungen beeinflussen, um das Signal in einer gewünschte Weise zu manipulieren.

Diese zusätzliche Funktion wird dazu genutzt, um den Benutzer zu einer bestimmten Verhaltensweise anzuregen. Insbesondere ist es bevorzugt, diese Funktion dazu zu nutzen, dem Benutzer ein gezieltes Entspannungstraining zukommen zu lassen. So kann der Benutzer während der Messdauer, die üblicherweise ohnehin für das bestimmen des Körperfettanteils notwendig ist, seinen Körper trainieren, um den zeitlichen Verlauf der physiologischen Messgröße zu ändern und so seinen Körper zu beeinflussen. Eine beispielhafte Anwendung dieser Möglichkeit ist ein gezieltes Entspannungstraining. Auch sonstige Trainingsmöglichkeiten sind möglich, beispielsweise kann der Benutzer über die Ermittlung des Schwerpunktes und dessen Bewegung angehalten werden, seinen Körper ruhig zu halten oder auch bestimmt Gymnastikbewegungen auszuführen.

Alle Ausgestaltung der Erfindung können bevorzugt über ein Trainingsprogramm gesteuert werden. Dieses Trainingsprogramm kann dem Benutzer, also der auf der Waage stehenden Person, gewisse Sollvorgaben machen, die er versuchen muss, während der Messperiode einzuhalten. Dies bedeutet, dass dem Benutzer angezeigt wird, wenn die Messgröße außerhalb eines der tolerablen Bereiches liegt. Gleiches gilt für den Schwerpunkt. Ferner kann neben einer reinen Bestimmung, ob die Messgröße außerhalb eines Grenzwertes liegt, auch ein zeitlicher Verlauf der Messgröße vorgegeben werden, so dass der Benutzer versuchen muss, seinen Körper dahingehend zu manipulieren, dass der tatsächliche gemessene Wert der Messgröße sich an diesem vorgegebenen Sollverlauf orientiert.

Im Falle einer besonders leicht herzustellenden Variante der Erfindung werden diejenigen Elektroden genutzt, die bei einer üblichen Körperfettanalysewaage ohnehin bereits vorhanden sind. Es sind dies die beiden Elektroden, die auf der Wägeplatte angeordnet sind und üblicherweise im Rahmen der Bestimmung des Körperfettanteils zur Messung des Widerstandes des Körpers genutzt werden. Üblicherweise wird im Rahmen der Bestimmung des Körperfettanteils nämlich von einer Elektrode ein Strom in den Fuß der auf der Wägeplatte stehenden Person eingekoppelt, der dann durch die Beine der Person zur gegenüberliegenden Elektrode verläuft, wobei der Widerstand des Körpers ein Maß für den Körperfettanteil ist.

Die Elektroden können dabei, wie bereits aus dem Stand der Technik bekannt, Metallelektroden, Teilbereiche einer metallischen Wägeplatte sein oder auch im Rahmen einer elektrisch leitfähigen Beschichtung, zum Beispiel einer so genannten ITO-Beschichtung, auf eine nicht leitfähige Wägeplatte aufgebracht sein.

Die elektrische Leitfähigkeit des menschlichen Körpers ist allerdings nicht nur von dem Körperfettanteil abhängig. Zusätzliche Einflussgrößen sind zum Beispiel Blutdruck oder Pulsfrequenz. Diese beiden Parameter sind jedoch vom Benutzer manipulierbar, so dass ihm, insbesondere in Verbindung mit einer Verhaltensanweisung, die Möglichkeit gegeben wird, über die Beobachtung der Abweichung des Ist-Wertes der Messgröße und dem gleichzeitigen Anzeigen eines Soll-Wertes diese Parameter in eine gewünschte Richtung zu ändern.

Beispielsweise kann die Person durch gezieltes Atemtraining den Blutdruck manipulieren. Auch die Pulsfrequenz ist dann änderbar. Nachdem der Messwert von Blutdruck und Pulsfrequenz abhängig ist, kann die auf der Wägeplatte stehende Person somit über eine Erfolgskontrolle gezielt und insbesondere optisch sichtbar den Blutdruck zum Beispiel durch eine regelmäßige, vertiefte Atmung manipulieren.

Die hierbei gewonnenen Erfahrungen werden insbesondere dadurch, dass der Trainingserfolg in der Anzeigevorrichtung durch Annähern des gemessenen Ist-Verlaufs der Messgröße an den geforderten Soll-Verlauf auch für physiologisch oder medizinisch nicht vorgebildete Benutzer unmittelbar sichtbar wird, für den Benutzer nachvollziehbar. Dies führt dazu, dass er gleiche Techniken nach einer gewissen Anzahl von Übungsperioden auch ohne die erfindungsgemäße Waage anwenden kann, beispielsweise um in Stresssituationen den Kreislauf gezielt zu beruhigen.

Besonders vorteilhaft ist es, wenn die erfindungsgemäße Waage zusätzliche Sensoren nutzt, um den physiologischen Zustand des Körpers der auf der Wägeplatte stehenden Person noch genauer ermitteln zu können.

Beispielhafte Möglichkeiten, um zusätzliche Körperfunktionen zu messen, bestehen zum Beispiel in der Messung der Muskelspannung. Auch kann der Widerstand bzw. die Leitfähigkeit der Haut über eine Widerstandsmessung als Messwert genutzt werden. Zusätzliche Messgrößen können die Kapazität der unteren Hautschichten des Fuß sein, wenn die Elektrode auf der Wägeplatte als erste Kondensatorplatte und die elektrisch leitfähige Schicht innerhalb des Fußes als zweite Kondensatorplatte innerhalb eines Stromkreises genutzt wird. Hier kann also ebenfalls ein Strom von der Wägeplatte über die Beine des Benutzers zu einer gegenüberliegenden Elektrode geführt werden, wobei die erste Elektrode dann allerdings als Teil eines Kondensators genutzt wird. Insbesondere in Verbindung mit Wechselspannungen kann hierüber ein zusätzliches Messergebnis erzielt werden. So ist es möglich, mit einem Elektrodensystem sowohl den ohmschen Widerstand als auch die Kapazität des Fußes zu nutzen.

Weitere Parameter, die Rückschlüsse auf den physiologischen Zustand des Körpers zu lassen, sind zum Beispiel die Durchblutung der Haut der Fußsohle. Hier kann in die Wägeplatte eine Lichtquelle integriert sein, wobei das abgegebene Licht auf den Fuß gerichtet ist. Diese Lichtquelle kann zum Beispiel ein kleiner, ungefährlicher Laser sein. Auch LEDs können hier verwendet werden. Die vom Fuß zurück reflektierte Streustrahlung kann dann erfasst und ausgewertet werden, wobei die Reflektionseigenschaften des Fuß ein Maß für die Durchblutung sind.

Weitere mögliche Messgrößen sind die Oberflächentemperatur der Fußsohle oder auch die Verteilung der räumlichen Belastung der Wägeplatte. Die Oberflächentemperatur der Fußsohle kann leicht über entsprechende Thermoelemente gemessen werden. Die räumliche Verteilung der Belastung der Wägeplatte kann zum Beispiel über Piezo-Folien ermittelt werden, die über die Wägeplatte hinweg segmentweise angeordnet sind und über die druckabhängige Änderung der Leitfähigkeit der einzelnen Segmente bestimmen können, an welchen Stellen der Wägeplatte welche Gewichtsanteile angekoppelt werden. Hierüber kann dann die Auswerteelektronik ermitteln, ob die auf der Wägeplatte stehende Person zum Beispiel dazu neigt, die Verse oder den Fußballen stärker zu belasten.

Schließlich ist es möglich, über die ohnehin vorhandenen Wägezellen zu ermitteln, wie sich der Schwerpunkt der auf der Wägeplatte stehenden Person verändert. Hier kann die Person entweder dazu angehalten werden, möglichst ruhig zu stehen, also den Schwerpunkt möglichst in einer Position zu halten. Alternativ kann die Person aber auch zu gezielten Bewegungslinien angeregt werden, so dass in der Anzeigevorrichtung die Schwerpunktbewegung vorgegeben wird, der die Person dann versuchen muss, zu folgen.

Insbesondere in Verbindung mit der Messung anderer physiologischer Größen kann der Person dann angezeigt werden, welchen Einfluss zum Beispiel eine ruhige, kreisförmige Körperbewegung auf den Blutdruck hat. Bevorzugt werden die verschiedenen Einflussfaktoren auf die körperlichen Werte miteinander kombiniert, so dass die Person zum Beispiel über gezieltes Atemtraining oder auch Bewegungstraining eine Rückmeldung hinsichtlich des Einflusses des Trainings auf ihren Körper erhält.

Die Ausgabevorrichtung kann unterschiedlichste Formen aufweisen, insbesondere können unterschiedliche Ausgabevorrichtung auch zu einer Gesamtvorrichtung kombiniert werden. Zunächst kommt als Ausgabevorrichtung natürlich das ohnehin an der Waage vorhandene Display in Betracht. Hier lässt sich einfach realisieren, dass der Messwert der Sensorik ausgegeben wird. Bevorzugt wird, damit der zeitliche Verlauf der Messgröße dargestellt werden kann, hier ein kleiner Bildschirm verwendet, der zur Kurvendarstellung genutzt werden kann.

Wie auch schon bei der eingangs genannten, bekannten Waage ist es ferner bevorzugt, das Display unabhängig von der Wägeplatte oder einem Gehäuse kabellos vorzusehen. Dies ermöglicht es, dass der Benutzer das Display im Auge behalten kann, ohne zum Beispiel den Körper entsprechend zu bewegen, so dass er das auf dem Boden befindliche Display erkennen kann. Insbesondere dann, wenn Schwerpunktbewegungen oder Belastungsverteilungen gemessen werden sollen, könnte ansonsten das Ablesen des Displays das Messergebnis beeinflussen.

Eine einfache Ausgestaltung einer mobilen Ausgabevorrichtung ist ein Display, das über Funkverbindungen, eine Bluetooth-Verbindung oder möglicherweise auch über ein flexibles Kabel mit der Auswerteelektronik der Personenwaage verbunden ist.

Darüber hinaus muss die Ausgabe auch nicht oder nicht ausschließlich visuell erfolgen. Weitere Ausgestaltung der Erfindung nutzen somit zum Beispiel Lautsprecher, um Informationen an die Person auf der Wägeplatte weiterzugeben. Diese Informationen können die Ausgabe der physiologischen Messgröße umfassen, wobei die Änderung der physiologischen Messgröße dann zum Beispiel durch die Frequenz eines Tones darstellbar ist.

Sofern über einen Lautsprecher mehrere physiologische Messgrößen ausgegeben werden sollen, kann dies dadurch realisiert werden, dass nacheinander Tonfolgen ausgegeben werden, wobei jede Tonfolge einer physiologischen Messgröße entspricht. Hier kann dann über die Höhe des jeweiligen Tones einer Tonfolge dem Benutzer angezeigt werden, in welchem Bereich die physiologische Messgröße sich derzeit befindet.

Ferner kann ein Lautsprecher auch genutzt werden, um bestimmte Grenzwerte anzugeben. So kann zum Beispiel immer dann, wenn der Schwerpunkt der Person sich außerhalb eines gewünschten Bereiches befindet, ein Signalton erklingen. Gleiches gilt auch für alle anderen physiologischen Messgrößen, so dass der Benutzer zum Beispiel zum Erreichen eines Idealwertes seines Körpers dafür Sorge tragen muss, dass kein Ton hörbar ist.

Weitere Ausgestaltungen der Erfindung verwenden Kombinationen von Ausgabevorrichtungen. Hier kann zum Beispiel das ohnehin an der Waage vorhandene Display, unabhängig davon, ob es an der Wägeplatte oder mobil zu angeordnet ist, zur Ausgabe eines ersten Messwertes genutzt werden. Weitere Ausgabevorrichtungen, in Displayform, als Lautsprecher oder auch in anderer Form, z.B. als Vibrationen erzeugendes Element, können dann hinzutreten.

So kann beispielsweise eine der Ausgabevorrichtungen von einem oder mehreren Leuchtbändern gebildet sein, die gegenüber der auf der Wägeplatte stehenden Personen angeordnet sind. Befindet sich die Person in einer gewünschten Körperhaltung kann beispielsweise das Leuchtband entweder die aktiviert oder grün ausgeleuchtet sein. Bewegt dagegen die Person außerhalb des gewünschten Bereiches kann auf der Seite, zu der die Person sich hingelegt hat, mit zunehmender Abweichung von der Soll-Position ein zunehmend intensiverer Rot-Ton oder mehrere rote LEDs aktiviert werden um der Person anzuzeigen, dass sie die Körperhaltung korrigieren sollte.

Eine weitere bevorzugte Ausgestaltung der Erfindung nutzt mobile Computer um das erfindungsgemäße System ohne zusätzlichen Hardwareaufwand umzusetzen. So kann beispielsweise ein übliches Notebook, ein Tablett-PC oder ein Smartphone genutzt werden, um einerseits die physiologische Messgröße bzw. die physiologischen Messgrößen anzuzeigen oder akustisch auszugeben.

Andererseits kann der in diesen Geräten vorhandene Prozessor die erforderlichen Berechnungen durchführen, so dass die Auswerteelektronik der Personenwaagen die Messgrößen über die üblichen, kabelgebunden war kabellosen Verbindungen auf diese Geräte übertragen kann, wobei dort dann ein Programm abläuft, dass die erforderlichen Funktionen umsetzt. Dies ist insbesondere das verknüpfen der physiologischen Messgröße mit einem Körperzustand, optional das Vergleichen der physiologischen Messgröße mit anderen Messgrößen eines anderen Sensors sowie die Errechnung, inwieweit die tatsächliche Messgröße von der gewünschten oder vorgegebenen Soll-Größe abweicht.

Nachdem insbesondere Smartphones und so genannte Tablett-PCs über alle notwendigen Komponenten verfügen, um die über die Sensorik der Waage hinausgehenden Aufgaben zu erfüllen, ist es bevorzugt, derartige Geräte zur Auswertung der Messgröße zu nutzen. Insbesondere wird es möglich, durch kleinere Programme, insbesondere so genannte APPs, die Messgröße auszuwerten und darzustellen. Dies bedeutet, dass die Erfindung besonders vorteilhaft umsetzbar ist, da lediglich die vorhandenen Waagen mit der Möglichkeit ergänzt werden müssen, die Messgrößen kontinuierlich oder in vorgegebenen Intervallen mit den Geräten auszutauschen. Alle übrigen Funktionen, die oben beschrieben wurden, können dann von diesen Geräten ausgeführt werden.

Da aber bereits Waagen, zum Beispiel die eingangs beschriebene bekannte Waage, vorhanden sind, die drahtlos mit anderen Geräten Daten austauschen können, muss zur Umsetzung der Erfindung auf Seiten der Waage lediglich ein kleines Programm angepasst werden, um die Auswerteelektronik zu veranlassen, den erforderlichen Datenaustausch durchzuführen. Alle übrigen Funktionen können dann auf Seiten des mobilen Computers umgesetzt werden.

Ein weiterer Vorteil einer drahtlosen Kommunikation zwischen einem mobilen Computer und den mit der erfindungsgemäßen Sensoren ausgestatteten Waagen besteht darin, dass diese Computer sich mit anderen Waagen koppeln können, so dass der Benutzer nicht darauf angewiesen ist, immer die gleiche Waage zu benutzen, um ein bestimmtes Ergebnis zu erzielen. Insbesondere können diese Geräte zusätzlich genutzt werden, um bestimmte Werte speichern und auch langfristig einen Gewichtsverlauf mit einem gewünschten Wert zu vergleichen.

So kann zum Beispiel das jeweilige Gewicht des Benutzers abgespeichert werden, um diesen in die Lage zu versetzen, eine langfristige Gewichts-und Erfolgskontrolle durchzuführen. Zusätzlich kann diese Speicherfunktion genutzt werden, um den Trainingserfolg hinsichtlich der Körperbeherrschung, so wie es oben beschrieben ist, zu dokumentieren und zu verbessern.

So kann es vorteilhaft sein, dass der Benutzer mit leichten Übungen anfängt, während er mit zunehmendem Lernfortschritt dann schwierigere Übungen absolviert. Insbesondere kann hierin der Lernerfolg der vorherigen Übungen einfließen und eine entweder nicht benötigte oder bereits erlernte Fähigkeit für den weiteren Funktionsumfang ausgeklammert werden.

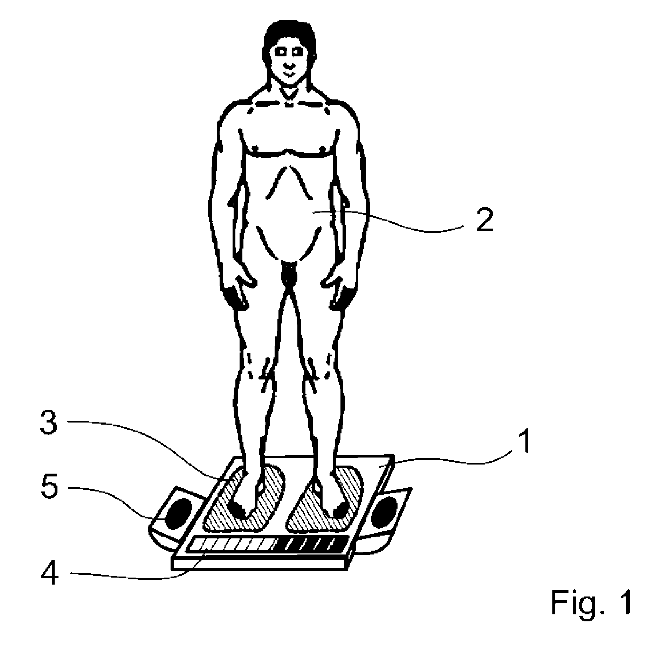

Weitere Merkmale und Vorteile der Erfindung ergeben sich aus den Unteransprüchen und der Beschreibung eines bevorzugten Ausführungsbeispiels der Erfindung anhand der Zeichnung in Figur 1.

### Kurze Beschreibung der Zeichnungsfiguren

In Figur 1 ist prinzipiell und schematisch eine Ausführungsform der Erfindung mit den erforderlichen Komponenten dargestellt.

### Beschreibung der Ausführungsarten

Grundsätzlich besteht die Erfindung aus der Verwendung einer handelsüblichen Waage zu neuen Trainings- und Übungseffekten, wobei vergleichsweise wenige Teile zur Umsetzung der Erfindung geändert werden müssen.

Der Benutzer 2 nutzt eine klassische Personenwaage mit auf der Wägeplatte 1 angeordneten physiologischen Sensoren 3, hier in Form von zwei unter den Füssen angeordneten Elektroden, um über die reine Gewichtsmessung hinaus weitere Körperdaten zu ermitteln. Zusätzlich wird die Änderung dieser Körperdaten über eine vorgegebene Zeitspanne analysiert und im Rahmen eines Biofeedbacks dem Benutzer 2 über eine optische Anzeigevorrichtung 4, hier als Balkenanzeige mit mittlerem Ausschlagpegel dargestellt, angezeigt.

Hier bietet sich insbesondere die Zeitdauer als Messperiode an, die für die Ermittlung des Körperfettanteils ohnehin notwendig ist, also eine Wägeperiode von etwa 30 Sekunden. Während dieser Zeit wird der Verlauf der gemessenen Daten ausgewertet und dem Benutzer möglicherweise zusätzlich über eine Trainingsanweisung mitgeteilt, wie er sich verhalten soll und welche Auswirkungen dies momentan auf die gemessenen Körperdaten hat.

Um den erfindungsgemäßen Zweck umsetzen zu können, ist zusätzlich zu den Grundbauteilen einer Körperfettanalysewaage, nämlich der Wägeplatte 1, die sich über Wägezellen auf den Boden abstützt, einer Auswerteelektronik und einer als Anzeige ausgebildeten Ausgabevorrichtung 4. Zusätzlich kann die Waage die in Figur 1 dargestellte akustische Ausgabevorrichtung 5 aufweisen, wobei je nach Übermittlung der Daten im Rahmen des Biofeedbacks an den Benutzer 2 die optische Ausgabevorrichtung 4 dann auch entfallen kann. Auch können beide oder eine der genannten Funktionen von anderen Geräten übernommen werden, die mit der Waage über eine Kabel oder die üblichen, kabellosen Verbindungen gekoppelt werden können.

Die Auswerteelektronik ist, entweder auf Seiten der Waage oder als externe Logik, so ausgebildet, dass sie den zeitlichen Verlauf einer physiologischen Messgröße ermittelt und auswertet. Hierzu kann bevorzugt zunächst als physiologischer Sensor 3 das Elektrodensystem genutzt werden, das jede Körperfettanalysewaage ohnehin aufweist.

Allerdings müssen nicht zwangsläufig die Elektroden für die erfindungsgemäße Umsetzung vorhanden sein oder genutzt werden. Vielmehr können auch weitere physiologische Sensoren 3 verwendet werden, wie zum Beispiel Temperaturfühler, Drucksensoren oder alle anderen Sensoren, die den Zustand der Person 2 anhand einer signifikanten Messgröße bestimmen können. Ferner kann die Schwerpunktsverteilung des Gewichtes auf der Wägeplatte 1 ermittelt und zum Training verwendet werden.

Die Auswerteelektronik der erfindungsgemäßen Waage bzw. des erfindungsgemäßen Systems aus einer Waage mit integriertem Biofeedbacksystem bzw. einer Waage mit Übermittlungsmöglichkeit für physiologischen Messgrößen an ein Auswertesystem zur Ermittlung des physiologischen Zustandes eines Körpers einer Person 2, die auf der Wägeplatte 1 steht, ermittelt anhand der gemessenen physiologischen Messgrößen den Zustand zumindest einer Eigenschaft des Körpers. Das Ergebnis dieser Ermittlung wird dem Benutzer über die Ausgabevorrichtung 4 angezeigt.

Der wesentliche Vorteil der Erfindung besteht nun darin, dass hier kein momentaner Ist-Zustand, sondern ein zeitlicher Verlauf ermittelt und angezeigt wird sowie insbesondere kontinuierlich die Änderung der Messgröße dem Benutzer 1 über die eine oder andere Weise, insbesondere die akustische Ausgabevorrichtung 5 oder eine visuelle Ausgabevorrichtung 4 angezeigt wird.

Besonders vorteilhaft ist es dabei, wenn dem Benutzer 1 nicht nur die Änderung der Messgröße mitgeteilt wird, sondern auch der Abstand zwischen der Messgröße und einem einzuhaltenden Soll-Wert, so dass der Benutzer 1 in die Lage versetzt wird, diesen Abstand zu verringern und so einen Trainingserfolg zu erzielen.

Insbesondere sofern eine akustische Ausgabevorrichtung 5 vorgesehen ist können dem Benutzer 2 auch weitere Informationen, wie beispielsweise Bewegungsanleitungen oder auch der Beginn und das Ende der Messperiode gegeben werden. Ferner kann beruhigende Musik zusätzlich einen Trainingserfolg fördern und das Verweilen auf der Personenwaage attraktiver gestalten.

### Liste der Bezugszeichen

1 Wägeplatte

2 Person

3 Physiologischer Sensor

4 Ausgabevorrichtung

5 Akustische Ausgabevorrichtung

## Patentansprüche

1. Personenwaage mit einer Wägeplatte (1) zur Aufnahme einer zu wiegenden Last einer auf der Wägeplatte stehenden Person (2), einer von mindestens einen physiologischen Sensor (3) gebildeten Sensorik zur Messung wenigstens einer physiologischen Messgröße des auf der Wägeplatte (1) stehenden Benutzers (2) und einer Auswerteelektronik, die anhand einer Deformation von Wägezellen nach einer Messperiode das Gewicht der Person zu bestimmen und über eine Ausgabevorrichtung auszugeben vermag, **dadurch gekennzeichnet, dass** die Auswerteelektronik die zeitliche Änderung der physiologischen Messgröße während der Messperiode über eine Ausgabevorrichtung (4) in einer Weise auszugeben vermag, dass die Person diese wahrzunehmen vermag, um die Person so in die Lage zu versetzen, durch die Wahrnehmung der zeitlichen Änderung der physiologischen Messgröße im Sinne einer Rückkopplung ihre, die physiologische Messgröße beeinflussenden Körperfunktionen bewusst zu beeinflussen.

2. Personenwaage nach Anspruch 1, **dadurch gekennzeichnet, dass** das die Wägeplatte (1) über zumindest drei, bevorzugt vier, Wägezellen abgestützt ist, wobei die Auswerteelektronik den Schwerpunkt der durch die auf der Wägeplatte stehenden Person (2) gebildeten Last anhand der unterschiedlichen Messergebnisse der einzelnen Wägezellen sowie den zeitlichen Verlauf der Schwerpunktbewegung zu bestimmen und über die Ausgabevorrichtung (4) auszugeben vermag.

3. Personenwaage nach Anspruch 1 oder 2, **dadurch gekennzeichnet, dass** die Ausgabevorrichtung (4) eine optische Anzeige zur Ausgabe des zeitlichen Verlaufs der physiologischen Messgröße oder der Schwerpunktbewegung umfasst.

4. Personenwaage nach einem der vorhergehenden Ansprüche, **dadurch gekennzeichnet, dass** die Ausgabevorrichtung (4) alternativ oder zusätzlich wenigstens eine akustische Ausgabevorrichtung (5), insbesondere in Form eines Lautsprechers zur akustischen Ausgabe des zeitlichen Verlaufs der physiologischen Messgröße oder der Schwerpunktbewegung und/oder des Beginns und Endes der Messperiode umfasst.

5. Personenwaage nach einem der beiden vorhergehenden Ansprüche, **dadurch gekennzeichnet, dass** die Ausgabevorrichtung (4) zusätzlich zum zeitlichen Verlauf der physiologischen Messgröße das Über- oder Unterschreiten eines Grenzwertes der physiologischen Messgröße oder, sofern mehrere physiologische Messgrößen bestimmt werden, einer oder mehrerer der physiologischen Messgrößen und/oder die Bewegung des Schwerpunktes über einen festgelegten Extremwert hinaus anzuzeigen vermag.

6. Personenwaage nach einem der vorhergehenden Ansprüche, **dadurch gekennzeichnet, dass** sie einen insbesondere an der Wägeplatte (1) angeordneten Vibrationserzeuger aufweist, über den die Auswerteelektronik den zeitlichen Verlauf der physiologischen Messgröße, den Verlauf der Schwerpunktbewegung und/oder das Überschreiten eines Grenzwertes für die physiologische Messgröße oder die Schwerpunktbewegung in Form von insbesondere an den Fußsohlen des Benutzers (1) wahrnehmbaren Vibrationen auszugeben vermag.

7. Personenwaage nach Anspruch 1, **dadurch gekennzeichnet, dass** die Sensorik als Sensor für die physiologische Messgröße einen Sensor zur Bestimmung des Hautleitwertes, einen Sensor zur Bestimmung der Temperatur der auf der Wägeplatte (1) stehenden Füße, einen Sensor zur Messung der Impedanz der auf der Wägeplatte (1) stehenden Person (2), einen Sensor zur Bestimmung der Muskelspannung und/oder einen Sensor zur Ermittlung der von den Füßen der auf der Wägeplatte (2) stehenden Person zurückreflektierten Streustrahlung einer Lichtquelle aufweist.

8. Personenwaage nach einem der vorhergehenden Ansprüche, **dadurch gekennzeichnet, dass** die Ausgabevorrichtung (4) ein mit einer Auswerteelektronik kabelgebunden oder drahtlos kommunizierendes Display aufweist.

9. Personenwaage nach dem vorhergehenden Anspruch, **dadurch gekennzeichnet, dass** das Display ein Display eines tragbaren Computers oder Smartphones ist.

10. Personenwaage nach einem der vorhergehenden Ansprüche, **dadurch gekennzeichnet, dass** die Ausgabevorrichtung (4) mehrere räumlich voneinander getrennte Anzeigen für die gemessenen Werte unterschiedlicher physiologischer Messgrößen und/oder die Ausgabe einer Messgröße sowie unabhängig hiervon die Ausgabe des Überschreitens eines Grenzwertes aufweist.
